# EUROPÄISCHE PATENTSCHRIFT

(11) **EP 1 470 778 B1**
(45) Veröffentlichungstag und Bekanntmachung des Hinweises auf die Patenterteilung: **21.05.2008**
(21) Anmeldenummer: 04000493.9
(22) Anmeldetag: 13.01.2004
(51) Int. Cl.: A61B 3/12

(54) **Vorrichtung zur Projektion eines Lichtstrahls**
Device for projecting a light beam
Dispositif de projection d'un faisceau lumineux

(30) Priorität: 25.04.2003 DE 20306542 U
(43) Veröffentlichungstag der Anmeldung: 27.10.2004
(73) Patentinhaber: Oculus Optikgeräte GmbH, 35582 Wetzlar-Dutenhofen (DE)
(72) Erfinder: Köst, Gert, 30171 Hannover (DE)
(74) Vertreter: von den Steinen, Axel

(56) Entgegenhaltungen:
- DE-A- 3 543 648
- DE-A- 4 015 920
- US-A- 2 222 937
- US-A- 4 125 320
- US-A- 4 168 126
- US-A- 5 742 426
- US-A- 5 815 242

## Beschreibung

Die Erfindung betrifft ein ophthalmologisches Gerät mit einer Vorrichtung zur Projektion eines Lichtstrahls auf ein Objekt nach dem Oberbegriff des Anspruchs 1.

Gattungsgemäße Geräte werden in der Medizintechnik verwendet, um Untersuchungen am menschlichen Auge durchführen zu können. Insbesondere in ophthalmologischen Scheimpflugkameras werden Projektionseinrichtungen benötigt, um einen Lichtstrahl auf das zu untersuchende Auge zu projizieren. Zur Erzeugung des Lichtstrahls ist bei gattungsgemäßen Vorrichtungen eine Lichtquelle vorhanden, in der durch Umwandlung beispielsweise elektrischer Energie ein gerichteter Lichtstrahl erzeugt werden kann. In welcher Weise die Projektionsoptik ausgebildet ist, ist grundsätzlich beliebig. Es sind dabei durchaus auch Vorrichtunggen denkbar, bei denen die Projektionsoptik keinerlei Umlenkeinrichtungen und Linsen enthält.

Für viele Anwendungen wird eine möglichst homogene Verteilung der Lichtintensität im Lichtstrahl gewünscht, da durch Inhomogenitäten der Lichtintensitäten die Messergebnisse in unerwünschter Weise verfälscht werden können. Nachteilig an bekannten Geräten ist es, dass die meisten verfügbaren Lichtquellen, beispielsweise Glühbirnen mit einer elektrischen Heizwendel oder aus mehreren Leuchtdioden zusammengesetzte Lichtquellen, eine relativ inhomogene Verteilung der Lichtintensität im Lichtstrahl aufweisen. Besteht die Lichtquelle beispielsweise aus mehreren Leuchtdioden, deren emittiertes Licht gemeinsam gebündelt den Lichtstrahl der Vorrichtung bildet, so wird von jeder einzelnen Leuchtdiode ein Leuchtintensitätsmaximum im Lichtstrahl verursacht.

Aus der US 4 125 320 ist ein Augenuntersuchungsgerät bekannt, bei dem als Lichtquelle ein Laser dient. Der zunächst scharf gebündelte Laserstrahl wird in der Projektionsoptik aufgespalten, um ein Interferenzlichtmuster auf das zu untersuchende Auge zu projizieren. Im Strahlengang des Laserstrahls ist ein beweglich gelagertes Prisma angeordnet, um durch Stellbewegungen des Prismas das Interferenzlichtmuster relativ zur Projektionsfläche im Auge zu bewegen. Durch die Bewegung des Interferenzlichtmusters kann der Nystagmus des Auges angeregt werden, solange das Auge eine Struktur des dargebotenen Interferenzlichtmusters erkennen kann. Auf diese Weise ist objektiv feststellbar, ob und gegebenenfalls bei welcher Struktur des Interferenzlichtmusters das untersuchte Auge das Muster noch erkennt.

Nachteilig an diesem Augenuntersuchungsgerät ist es, dass zur Erzeugung eines in seiner Lichtintensität sehr homogenen Lichtstrahls eine sehr aufwendige Lichtquelle, nämlich ein Helium-Neon-Laser, eingesetzt werden muss.

Ausgehend von diesem Stand der Technik ist es Aufgabe der vorliegenden Erfindung, ein ophthalmologisches Gerät vorzuschlagen, mit der die Lichtintensität im von der Lichtquelle erzeugten Lichtstrahl mit einfachen Mitteln homogenisiert werden kann.

Diese Aufgabe wird durch ein Gerät nach der Lehre des Anspruchs 1 gelöst.

Vorteilhafte Ausführungsformen der Erfindung sind Gegenstand der Unteransprüche.

Die Erfindung beruht auf dem Grundgedanken, Lichtquellen zu verwenden, die einen ungefähr linienförmigen Lichtstrahl abgeben. Unter einem linienförmigen Lichtstrahl soll dabei ein Lichtstrahl verstanden werden, dessen Ausbreitung quer zur Strahlrichtung ungefähr linienförmig ist. Solche linienförmigen Lichtquellen weisen Inhomogenitäten in der Lichtintensität auf, die beispielsweise von der verwendeten Leuchtwendel oder der Anordnung von mehreren Leuchtdioden nebeneinander herrührt. Diese Inhomogenitäten können erfindungsgemäß durch die Brechung im bewegten Prisma abgemildert oder gänzlich vermieden werden.

Beim Durchgang durch ein Prisma wird der Lichtstrahl parallel versetzt. Dazu weist das Prisma zumindest zwei im Wesentlichen planparallele Flächen auf. Beim Eintritt des Lichtstrahls an der ersten Fläche des Prismas wird der Lichtstrahl um einen bestimmten Winkel abgelenkt, wobei der Betrag dieser Ablenkung von der Stellung zwischen dem Lichtstrahl und der Fläche des Prismas abhängt. Beim Austritt des Lichtstrahls aus dem Prisma an der zweiten Fläche wird der Lichtstrahl wiederum um einen Winkel abgelenkt. Aufgrund der Planparallelität der beiden Flächen des Prismas ergibt sich, dass die Ablenkung des Lichtstrahls an den beiden Flächen genau gegengleich groß ist, so dass sich im Ergebnis eine parallele Versetzung des Lichtstrahls zwischen Eingangspunkt und Ausgangspunkt ergibt.

Da das Prisma beweglich gelagert ist und mittels einer Antriebseinrichtung angetrieben wird, wird der Lichtstrahl aufgrund der permanenten Änderung der relativen Lage zwischen Prisma und Lichtstrahl ständig innerhalb eines bestimmten Schwankungsbereichs verschoben. Die Verschiebung des Lichtstrahls erfolgt dabei mit einer Geschwindigkeit, die von der Bewegungsgeschwindigkeit des Prismas abhängt.

Im Ergebnis führt die erfindungsgemäße Durchleitung des Lichtstrahls durch ein bewegtes Prisma mit zumindest zwei planparallelen Flächen dazu, dass die Lichtintensitätsmaxima im Lichtstrahl ständig hin und her wandern und auf diese Weise gleichmäßig im Lichtstrahl verteilt werden. Werden beispielsweise Fotoaufnahmen des mit einem solchen Lichtstrahl beleuchteten Objekts gemacht, und liegt die Belichtungszeit in einem Zeitbereich, innerhalb dessen die Lichtintensitätsmaxima bereits mehrfach hin und her gewandert sind, so können die Lichtintensitätsmaxima auf der Fotoaufnahme nicht mehr unterschieden werden. Auf diese Weise erhält der Bediener also eine Fotoaufnahme mit absolut homogenen Beleuchtungsbedingungen.

Grundsätzlich ist es zur Bildung einer erfindungsgemäßen Vorrichtung ausreichend, wenn das verwendete Prisma genau zwei im Wesentlichen planparallele Flächen aufweist. Um die einsetzbare Bewegungskinematik beim Antrieb des Prismas vereinfachen zu können, ist es besonders vorteilhaft, wenn das Prisma mehrere Paare von jeweils im Wesentlichen planparallel einander zugeordneten Flächen aufweist. Insbesondere ist der Einsatz von Prismen denkbar, deren Flächen gleichmäßig über den Umfang des Prismas verteilt angeordnet sind und dabei ein gleichmäßiges n-Eck bilden. Auf diese Weise kann das Prisma dann zur Homogenisierung der Lichtintensität im Lichtstrahl in einfacher Weise rotatorisch angetrieben werden, wobei eine Drehrichtungsumkehr nicht erforderlich ist.

Eine besonders einfache Bauweise des verwendeten Prismas ergibt sich, wenn dieses vier Flächen zur Brechung des Lichtstrahls aufweist, wobei jeweils zwei Flächen planparallel angeordnet sind.

Um eine ausreichende Homogenisierung der Lichtintensität im Lichtstrahl beim Auftreffen des Lichtstrahls auf dem Objekt zu erreichen, sollte das Prisma eine bestimmte Mindestgeschwindigkeit nicht unterschreiten. Hat das Prisma beispielsweise vier Flächen zur Lichtbrechung, so ist es besonders vorteilhaft, wenn das Prisma mit einer Geschwindigkeit von zumindest ungefähr 100 Umdrehungen pro Sekunde angetrieben wird. Bei einer Belichtungsgeschwindigkeit von 50 Bildern pro Sekunde bedeutet dies, dass das Prisma während einer Bildaufnahme zwei Umdrehungcn durchläuft, so dass der Lichtstrahl während einer Bildaufnahme durch die vier Flächen des Prismas insgesamt acht Mal zwischen den maximalen Auslenkungen parallel verschoben worden ist. Eine Lokalisierung von lokalen Lichtintensitätsmaxima ist in den Einzelaufnahmen dann nicht mehr möglich.

Zum Antrieb des Prismas kann ein Antriebsmotor, insbesondere ein Elektromotor, vorgesehen sein. Soweit erforderlich, können zwischen Prisma und Antriebsmotor auch Übersetzungsgetriebe angeordnet werden.

Nach einer bevorzugten Ausführungsform der Erfindung können im Strahlengang hintereinander auch mehrere beweglich gelagerte und antreibbare Prismen angeordnet sein. Soweit die Prismen den Lichtstrahl dabei in einer Ebene parallel versetzen, kann dadurch die Homogenisierung der Lichtintensität verbessert werden.

Nach einer weiteren Ausführungsform sind die hintereinander angeordneten Prismen jeweils um eine Rotationsachse drehbar gelagert, wobei die Rotationsachsen der verschiedenen Prismen im Wesentlichen rechtwinkelig zueinander verlaufen. Im Ergebnis wird dadurch erreicht, dass der Lichtstrahl am ersten Prisma in einer ersten Ebene parallel versetzt wird und am zweiten Prisma in einer dazu rechtwinkelig verlaufenden Ebene parallel versetzt wird. Bei Benutzung einer Lichtquelle, die einen ungefähr punktförmigen Lichtstrahl abgibt, beispielsweise einer Laserlichtquelle, kann auf diese Weise der Laserlichtstrahl auf ein rechtwinkliges Feld abgebildet werden.

Wird als Lichtquelle eine Reihe von nebeneinander angeordneten Leuchtmitteln, beispielsweise Leuchtdioden, verwendet, so ist es besonders vorteilhaft, wenn der Lichtstrahl im Prisma um einen Betrag versetzt werden kann, der größer ist als der Abstand zwischen den jeweils benachbarten Leuchtmitteln. Im Ergebnis führt dies dazu, dass die Leuchtintensitätsmaxima während der Bewegung des Prismas soweit versetzt werden, dass die Versatzbereiche einander überschneiden.

In welcher Anwendungstechnik die erfindungsgemäße Vorrichtung eingesetzt wird, ist grundsätzlich beliebig. Besonders große Vorteile bietet die erfindungsgemäße Vorrichtung an Spaltprojektoren, wie sie beispielsweise auch in Scheimpflugkameras zum Einsatz kommen. Aber auch an anderen Geräten zur Durchführung von Untersuchungen am menschlichen Auge ist der Einsatz der erfindungsgemäßen Vorrichtung gut denkbar, da bei diesen Geräten grundsätzlich eine möglichst homogene Beleuchtung erwünscht ist.

Verschiedene Ausführungsformen der Erfindung sind in den Zeichnungen schematisch dargestellt und werden nachfolgend beispielhaft erläutert.

Es zeigen:
- **Fig. 1**: den prinzipiellen Aufbau einer ersten Ausführungsform in Ansicht von oben;
- **Fig. 2**: den prinzipiellen Aufbau einer zweiten Ausführungsform in Ansicht von oben.

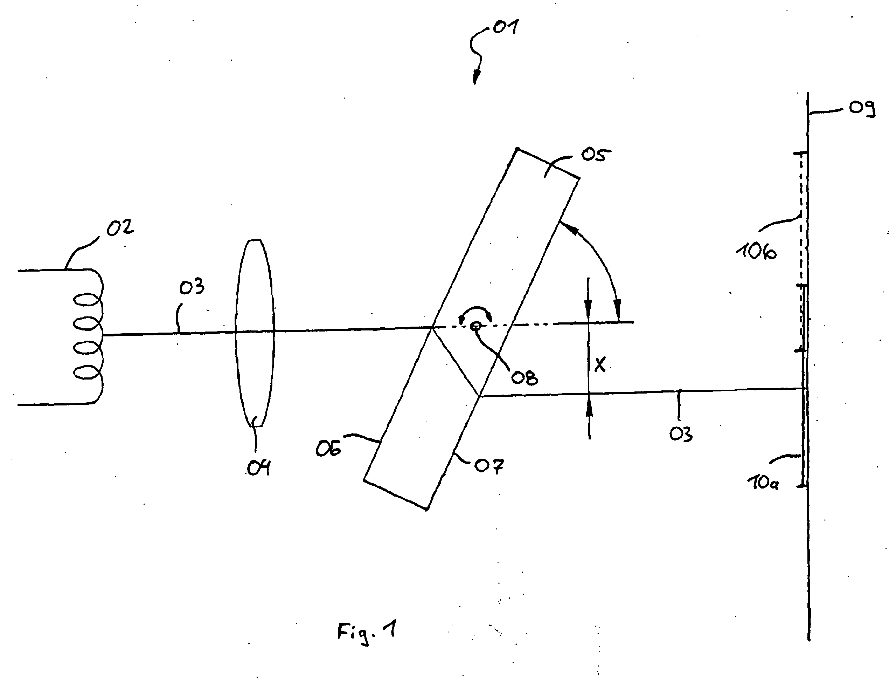

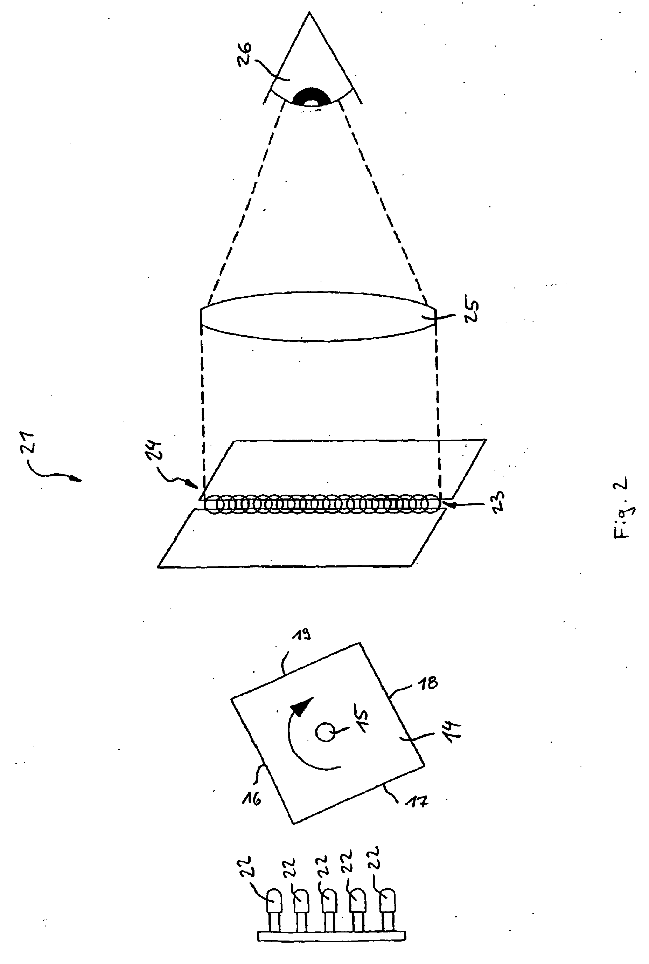

In **Fig. 1** ist eine erste Ausführungsform 01 einer erfindungsgemäßen Vorrichtung schematisch dargestellt. Mittels einer in der Art einer Leuchtwendel ausgebildeten Lichtquelle 02 wird ein im Wesentlichen linienförmiger Lichtstrahl, dessen Ausdehnung ungefähr der Länge der Leuchtwendel 02 entspricht, erzeugt. Zum leichteren Verständnis ist vom durch die Lichtquelle 02 erzeugten Lichtstrahl in **Fig. 1** lediglich die mittlere Strahlachse eingezeichnet, die den Lichtstrahl 03 repräsentieren soll. Auch ansonsten ist die optische Abbildung der Lichtstrahlen in **Fig. 1** und **Fig. 2** nur schematisch zu verstehen.

Nachdem der Lichtstrahl 03 eine Linse 04 durchlaufen hat, trifft er auf ein Prisma 05 mit zwei planparallel zueinander angeordneten Flächen 06 und 07, an denen der Lichtstrahl 03 aufgrund der unterschiedlichen optischen Dichte gebrochen wird. Aufgrund der Planparallelität der beiden Flächen 06 und 07 wird der Lichtstrahl 03 dabei um einen Betrag X parallel seitlich versetzt. Das Maß, um das der Lichtstrahl 03 seitlich versetzt wird, hängt dabei vom Winkel α ab, mit dem das Prisma 05 relativ zur Strahlachse des Lichtstrahls 03 angewinkelt ist.

Das Prisma 05 ist um eine Rotationsachse 08 drehbar gelagert und kann mittels eines nicht dargestellten Antriebs alternierend im Uhrzeigersinn und im Gegenuhrzeigersinn angetrieben werden. Im Ergebnis schwingt das Prisma 05 also um eine Mittellage, in der der Lichtstrahl 03 das Prisma 05 ohne Ablenkung durchläuft. Der in **Fig. 1** dargestellte Auslenkungswinkcl α stellt die Maximalposition des Prismas 05 im Uhrzeigersinn dar.

Nach Durchlaufen des Prismas 05 trifft der Lichtstrahl 03 auf eine Projektionsfläche 09 und bildet dort die Lichtquelle 02 als einen Lichtstreifen 10 ab. Der als durchgezogene Linie angedeutete Lichtstreifen 10a entspricht dabei der Abbildung der Lichtquelle 02 bei Stellung des Prismas 05 mit Anstellwinkel α in der Maximalposition gemäß **Fig. 1****.** Die Länge des Lichtstreifens 10a entspricht dabei der Länge der Lichtquelle 02 nach Abbildung durch die Sammellinse 04. Weiter ist in **Fig. 1** ein Lichtstreifen 10b strichliniert angedeutet. Der Lichtstreifen 10b entspricht der Abbildung der Lichtquelle 02 bei Stellung des Prismas 05 in der Maximalstellung im Gegenuhrzeigersinn (in **Fig. 1** nicht dargestellt). Man erkennt, dass der Lichtstreifen 10b im Vergleich zum Lichtstreifen 10a auf der Projektionsfläche 09 nach oben versetzt ist. Wird das Prisma 05 nun während des Betriebes der Vorrichtung 01 permanent zwischen den beiden Endstellungen auf und ab bewegt, so wandert der auf die Projektionsfläche 09 abgebildete Lichtstreifen 10 zwischen den Extrempositionen 10a und 10b. Im Überlappungsbereich zwischen den Lichtstreifen 10a und 10b werden auf diese Weise Inhomogenitäten der Lichtintensitätsverteilung im Lichtstrahl 03 homogenisiert.

In **Fig. 2** ist eine zweite Ausführungsform 21 einer erfindungsgemäßen Vorrichtung dargestellt, die in der Art eines Spaltprojektors ausgebildet ist, wie er beispielsweise in einer Scheimpflugkamera zur Untersuchung des menschlichen Auges eingesetzt werden kann. Als Lichtquelle der Vorrichtung 01 dienen fünf Leuchtdioden 22, die zueinander beabstandet nebeneinander in einer Linie angeordnet sind und einen linienförmigen Lichtstrahl abgeben, dessen Ausdehnung quer zur Strahlachse in Richtung des Spalts 23 in der Spaltblende 24 verläuft.

Im Strahlengang der von den Leuchtdioden 22 abgegebenen Lichtstrahlen ist das Prisma 14 angeordnet und wird im Uhrzeigersinn mit einer Umlaufgeschwindigkeit von zumindest 100 Umdrehungen pro Minute angetrieben. Durch das Prisma 14 wird das von den Leuchtdioden 22 abgegebene Licht jeweils seitlich parallel versetzt, so dass die Leuchtdioden 22 im Spalt 23 der Spaltblende 24 mehrfach abgebildet werden, wie dies in **Fig. 2** schematisch angedeutet ist. Die Leuchtintensitätsmaxima der verschiedencn Leuchtdioden 22 überlappen dabei, so dass insgesamt eine Homogenisierung der Leuchtintensitätsverteilung des Lichtstrahls im Spalt 23 erreicht wird. Dieser Lichtstrahl mit homogenisierter Leuchtintensitätsverteilung wird dann über eine Linse 25 in ein zu untersuchendes Auge 26 projiziert, um auf diese Weise Aufnahmen mit einer in **Fig. 2** nicht dargestellten Scheimpflugkamera machen zu können.

## Patentansprüche

1. Ophthalmologisches Gerät zur Durchführung von Untersuchungen am menschlichen Auge (26), mit einer Vorrichtung (01, 21) zur Projektion eines Lichtstrahls (03) auf das Auge (09, 26), wobei die Vorrichtung (01, 21) mit einer Lichtquelle (02, 22) zur Erzeugung des Lichtstrahls (03) und mit einer Projektionsoptik zur Weiterleitung des Lichtstrahls (03) von der Lichtquelle (02, 22) zum Auge (09, 26) ausgestattet ist, und wobei als Teil der Projektionsoptik im Strahlengang des Lichtstrahls (03) zwischen Lichtquelle (02, 22) und Auge (09, 26) zumindest ein Prisma (05, 14) mit zumindest zwei im Wesentlichen planparallelen Flächen (06, 07, 16, 17, 18, 19) angeordnet ist, und wobei das Prisma (05, 14) beweglich gelagert ist und mittels einer Antriebseinrichtung derart angetrieben werden kann, dass der Lichtstrahl (03) beim Durchgang durch die planparallelen Flächen (06, 07, 16, 17, 18, 19) des Prismas (05) abhängig von der Stellung des Prismas (05) um einen Betrag (X) parallel versetzt wird,
**dadurch gekennzeichnet,**
**dass** die Lichtquelle (02, 22) einen sich quer zur Strahlrichtung linienförmig ausbreitenden Lichtstrahl abgibt, wobei der Lichtstrahl eine inhomogene Verteilung der Lichtintensität aufweist, und wobei der linienförmige Lichtstrahl durch die Bewegung des Prismas (05, 14) unter Bildung eines wiederum linienförmigen Lichtstrahls in seine Längsrichtung versetzt wird.

2. Qphthalmologisches Gerät nach Anspruch 1,
**dadurch gekennzeichnet,**
**dass** das Prisma (14) mehrere Paare von jeweils im Wesentlichen planparallel einander zugeordneten Flächen (16, 17, 18, 19) aufweist, insbesondere dass das Prisma (14) in der Art eines Polygonprismas ausgebildet ist.

3. Ophthalmologisches Gerät nach Anspruch 2,
**dadurch gekennzeichnet,**
**dass** die planparallel einander zugeordneten Flächen (16, 17, 18, 19) gleichmäßig über den Umfang des Prismas (14) verteilt angeordnet sind und ein gleichmäßiges n-Eck bilden.

4. Ophthalmologisches Gerät nach Anspruch 2 oder 3,
**dadurch gekennzeichnet,**
**dass** das Prisma (14) zwei Paare von planparallel einander zugeordneten Flächen (16, 17, 18, 19) aufweist, insbesondere dass das Prisma (14) in der Art eines Würfelprismas ausgebildet ist.

5. Ophthalmologisches Gerät nach einem der Ansprüche 2 bis 4,
**dadurch gekennzeichnet,**
**dass** die verschiedenen, planparallel einander zugeordneten Flächen (16, 17, 18, 19) des Prismas (14) jeweils im Wesentlichen gleiche Abmessungen aufweisen.

6. Ophthalmologisches Gerät nach einem der Ansprüche 1 bis 5,
**dadurch gekennzeichnet,**
**dass** das Prisma (05, 14) um eine Rotationsachse (08, 15) schwenk- und/oder drehbar gelagert ist.

7. Ophthalmologisches Gerät nach Anspruch 6,
**dadurch gekennzeichnet,**
**dass** die Rotationsachse (08, 15) parallel zu den durch die planparallel einander zugeordneten Flächen (06, 07, 16, 17, 18, 19) des Prismas definierten Ebenen verläuft.

8. Ophthalmologisches Gerät nach Anspruch 6 oder 7,
**dadurch gekennzeichnet,**
**dass** das Prisma (14) umlaufend im Uhrzeigersinn oder im Gegenuhrzeigersinn angetrieben werden kann.

9. Ophthalmologisches Gerät nach Anspruch 8,
**dadurch gekennzeichnet,**
**dass** das Prisma (14) mit einer Geschwindigkeit von zumindest ungefähr 100 Umdrehungen pro Sekunde angetrieben werden kann.

10. Ophthalmologisches Gerät nach einem der Ansprüche 1 bis 9,
**dadurch gekennzeichnet,**
**dass** das Prisma (05, 14) mit einem Antriebsmotor, insbesondere mit einem Elektromotor, antreibbar ist.

11. Ophthalmologisches Gerät nach einem der Ansprüche 1 bis 10,
**dadurch gekennzeichnet,**
**dass** in der Vorrichtung mehrere beweglich gelagerte und antreibbare Prismen hintereinander im Strahlengang angeordnet sind.

12. Ophthalmologisches Gerät nach Anspruch 11,
**dadurch gekennzeichnet,**
**dass** die hintereinander angeordneten Prismen jeweils um eine Rotationsachse drehbar gelagert sind, wobei die Rotationsachsen der verschiedenen Prismen im Wesentlichen rechtwinkelig zueinander verlaufen.

13. Ophthalmologisches Gerät nach einem der Ansprüche 1 bis 12,
**dadurch gekennzeichnet,**
**dass** als Lichtquelle ein Leuchtmittel mit elektrisch beheizter Leuchtwendel (02) verwendet wird.

14. Ophthalmologisches Gerät nach einem der Ansprüche 1 bis 12,
**dadurch gekennzeichnet,**
**dass** als Lichtquelle mehrere in einer Linie nebeneinander angeordnete Leuchtmittel, insbesondere Leuchtdioden (22), verwendet werden.

15. Ophthalmologisches Gerät nach Anspruch 14,
**dadurch gekennzeichnet,**
**dass** der Lichtstrahl um einen Betrag versetzt werden kann, der größer ist als der Abstand zwischen jeweils benachbarten Leuchtmitteln (22).

16. Ophthalmologischcs Gerät nach einem der Ansprüche 1 bis 15,
**dadurch gekennzeichnet,**
**dass** die Vorrichtung (21) in der Art eines Spaltprojektors mit einer Spaltblende (24) ausgebildet ist, wobei der Lichtstrahl vom Prisma (14) in Spaltlängsrichtung versetzt wird.

17. Ophthalmologisches Gerät nach einem der Ansprüche 1 bis 16,
**dadurch gekennzeichnet,**
**dass** das Gerät in der Art einer ophthalmologischen Scheimpflugkamera ausgebildet ist.

## Claims

1. An ophthalmological instrument to carry out examinations of the human eye (26), with a device (01, 21) for projecting a light beam (03) onto the eye (09, 26), with the device (01, 21) being equipped with a light source (02, 22) for generating the light beam (03), and with projection optics for transmitting the light beam (03) from the light source (02, 22) to the eye (09, 26), and wherein at least one prism (05, 14) with at least two essentially plane-parallel surfaces (06, 07, 16, 17, 18, 19) is arranged in the beam path of the light beam (03) between the light source (02, 22) and the eye (09, 26) as part of the projection optics, and wherein the prism (05, 14) is movably supported and can be driven by means of a drive arrangement such that the light beam (03) is shifted in a parallel fashion by an amount (X) that depends on the position of the prism (05) when the light beam passes through the plane-parallel surfaces (06, 07, 16, 17, 18, 19) of the prism (05),
**characterized in that**
the light source (02, 22) emits a light beam spreading in a linear manner transversely to the beam direction, wherein the light beam has a non-homogeneous distribution of the light intensity, and wherein the linear light beam is shifted in its longitudinal direction by the movement of the prism (05, 14), forming a light beam which is, again, linear.

2. The ophthalmological instrument according to Claim 1,
**characterized in that**
the prism (14) has several pairs of surfaces (16, 17, 18, 19) which are respectively associated with each other in a substantially plane-parallel manner, in particular that the prism (14) is formed in the manner of a polygonal prism.

3. The ophthalmological instrument according to Claim 2,
**characterized in that**
the surfaces (16, 17, 18, 19) which are associated in a plane-parallel manner to each other, are arranged distributed uniformly over the periphery of the prism (14) and form a uniform n-gon.

4. The ophthalmological instrument according to Claim 2 or 3,
**characterized in that**
the prism (14) has two pairs of surfaces (16, 17, 18, 19) associated with each other in a plane-parallel manner, in particular that the prism (14) is formed in the manner of a cubic prism.

5. The ophthalmological instrument according to any of Claims 2 to 4,
**characterized in that**
the various surfaces (16, 17, 18, 19) of the prism (14) which are associated with each other in a plane-parallel manner respectively have substantially the same dimensions.

6. The ophthalmological instrument according to any of Claims 1 to 5,
**characterized in that**
the prism (05, 14) is mounted so as to be swivellable and/or rotatable about a rotation axis (08, 15).

7. The ophthalmological instrument according to Claim 6,
**characterized in that**
the rotation axis (08, 15) runs parallel to the planes defined by the surfaces (06, 07, 16, 17, 18, 19) of the prism which are associated with each other in a plane-parallel manner.

8. The ophthalmological instrument according to Claim 6 or 7,
**characterized in that**
the prism (14) can be driven rotating in a clockwise or anticlockwise direction.

9. The ophthalmological instrument according to Claim 8,
**characterized in that**
the prism (14) can be driven at a speed of at least approximately 100 revolutions per second.

10. The ophthalmological instrument according to any of Claims 1 to 9,
**characterized in that**
the prism (05, 14) is able to be driven by a driving motor, in particular by an electric motor.

11. The ophthalmological instrument according to any of Claims 1 to 10,
**characterized in that**
in the device, several movably mounted and drivable prisms are arranged one behind the other in the beam path.

12. The ophthalmological instrument according to Claim 11,
**characterized in that**
the prisms, arranged one behind the other, are respectively mounted rotatably about a rotation axis, wherein the rotation axes of the various prisms run substantially at right angles to each other.

13. The ophthalmological instrument according to any of Claims 1 to 12,
**characterized in that**
a light means with an electrically heated light filament (02) is used as the light source.

14. The ophthalmological instrument according to any of Claims 1 to 12,
**characterized in that**
several light means, arranged in a line adjacent to each other, in particular light-emitting diodes (22), are used as the light source.

15. The ophthalmological instrument according to Claim 14,
**characterized in that**
the light beam can be shifted by an amount which is greater than the distance between respectively adjacent light means (22).

16. The ophthalmological instrument according to any of Claims 1 to 15,
**characterized in that**
the device (21) is constructed in the manner of a slit projector with a slit diaphragm (24), wherein the light beam is shifted by the prism (14) in the longitudinal direction of the slit.

17. The ophthalmological instrument according to any of Claims 1 to 16,
**characterized in that**
the instrument is constructed in the manner of an ophthalmological Scheimpflug camera.

## Revendications

1. Instrument ophtalmologique pour procéder à des examens sur l'oeil humain (26), avec un dispositif (01, 21) pour la projection d'un faisceau lumineux (03) sur l'oeil (09, 26), le dispositif (01, 21) étant équipé d'une source lumineuse (02, 22) pour la création du faisceau lumineux (03) et avec une optique de projection, pour la retransmission du faisceau lumineux (03) de la source lumineuse (02, 22) vers l'oeil (09, 26), et un prisme (05, 14) avec au moins deux surfaces (06, 07, 16, 17, 18, 19) sensiblement à faces planes parallèles étant disposé en tant qu'élément de l'optique de projection, dans la trajectoire de faisceau du faisceau lumineux (03), entre la source lumineuse (02, 22) et l'oeil (09, 26) et le prisme (05, 14) étant logé de façon mobile et entraîné au moyen d'un dispositif d'entraînement, de sorte que lors du passage à travers les surfaces à faces planes parallèles 06, 07, 16, 17, 18, 19) du prisme (05) (en fonction de la position du prisme (05)), le faisceau lumineux (03) soit déplacé à la parallèle d'une valeur (X), **caractérisé en ce que**,
la source lumineuse (02, 22) délivre un faisceau lumineux se propageant sous forme linéaire, à la transversale de la direction du faisceau, le faisceau lumineux présentant une distribution inhomogène de l'intensité lumineuse et le faisceau lumineux de forme linéaire étant déporté dans sa direction longitudinale, par le déplacement du prisme (05, 14) en formant un faisceau lumineux de nouveau de forme linéaire.

2. Instrument ophtalmologique selon la revendication 1, **caractérisé en ce que**,
le prisme (14) comporte plusieurs paires de surfaces (16, 17, 18, 19) mutuellement associées sensiblement sur à faces planes parallèles, notamment **en ce que** le prisme (14) est réalisé à la manière d'un prisme polygonal.

3. Instrument ophtalmologique selon la revendication 2, **caractérisé en ce que**,
les surfaces (16, 17, 18, 19) mutuellement associées à faces planes parallèles sont disposées en distribution régulière sur la périphérie du prisme (14) et forment un n-polygone régulier.

4. Instrument ophtalmologique selon la revendication 2 ou 3, **caractérisé en ce que**,
le prisme (14) comporte deux paires de surfaces (16, 17, 18, 19) mutuellement associées à faces planes parallèles, notamment **en ce que** le prisme (14) est réalisé à la manière d'un prisme cubique.

5. Instrument ophtalmologique selon l'une quelconque des revendications 2 à 4, **caractérisé en ce que**,
les différentes surfaces (16, 17, 18, 19) mutuellement associées à faces planes parallèles du prisme (14) présentent chacune des dimensions sensiblement identiques.

6. Instrument ophtalmologique selon l'une quelconque des revendications 1 à 5, **caractérisé en ce que**,
le prisme (05, 14) est logé en étant susceptible de pivoter et/ou de tourner autour d'un axe de rotation (08, 15).

7. Instrument ophtalmologique selon la revendication 6, **caractérisé en ce que**,
l'axe de rotation (08, 15) s'étend à la parallèle des plans définis par les surfaces (06, 07, 16, 17, 18, 19) mutuellement associées à faces planes parallèles du prisme.

8. Instrument ophtalmologique selon la revendication 6 ou 7, **caractérisé en ce que**,
le prisme (14) est susceptible d'être entraîné en révolution dans le sens horaire ou dans le sens anti-horaire.

9. Instrument ophtalmologique selon la revendication 8, **caractérisé en ce que**,
le prisme (14) est susceptible d'être entraîné à une vitesse d'au moins environ 100 tours par seconde.

10. Instrument ophtalmologique selon l'une quelconque des revendications 1 à 9, **caractérisé en ce que**,
le prisme (05, 14) est susceptible d'être entraîné à l'aide d'un moteur d'entraînement, notamment à l'aide d'un moteur électrique.

11. Instrument ophtalmologique selon l'une quelconque des revendications 1 à 10, **caractérisé en ce que**,
dans le dispositif, plusieurs prismes logés de façon mobile et susceptibles d'être entraînés sont disposés dans la trajectoire du faisceau.

12. Instrument ophtalmologique selon la revendication 11, **caractérisé en ce que**,
les prismes disposés les uns derrière les autres sont logés chacun de façon rotative autour d'un axe de rotation, les axes de rotation des différents prismes s'étendant sensiblement à angle droit les uns par rapport aux autres.

13. Instrument ophtalmologique selon l'une quelconque des revendications 1 à 12, **caractérisé en qu'**un moyen d'éclairage avec filament lumineux (02) à chauffage électrique est utilisé en tant que source lumineuse.

14. Instrument ophtalmologique selon l'une quelconque des revendications 1 à 12, **caractérisé en ce que**,
plusieurs moyens d'éclairage (22) disposés côte à côte sur une ligne, notamment des diodes électroluminescentes sont utilisés en tant que source lumineuse.

15. Instrument ophtalmologique selon la revendication 14, **caractérisé en ce que**,
le faisceau lumineux est susceptible d'être déporté d'une valeur qui est supérieure à l'écart entre des moyens d'éclairage (22) chaque fois voisins.

16. Instrument ophtalmologique selon l'une quelconque des revendications 1 à 15, **caractérisé en ce que**,
le dispositif (21) est réalisé à la manière d'un projecteur à fente, avec un diaphragme à fente (24), le faisceau lumineux étant déporté par le prisme (14) en direction longitudinale de la fente.

17. Instrument ophtalmologique selon l'une quelconque des revendications 1 à 16, **caractérisé en ce que**
l'instrument est réalisé à la manière d'une caméra ophtalmologique à temps de vol.
